# EUROPEAN PATENT APPLICATION

(11) **EP 4 651 632 A1**
(43) Date of publication of application: **19.11.2025**
(21) Application number: 25173836.5
(22) Date of filing: 01.05.2025
(51) Int. Cl.: H05H 7/02

(54) **VACUUM AND HIGH PRESSURE RF SYSTEM FOR MEDICAL LINEAR ACCELERATORS**

(30) Priority: 15.05.2024 US 202418665081
(71) Applicant: Varian Medical Systems, Inc., Palo Alto, CA 94304 (US)
(72) Inventor: VAN HETEREN, John, Foster City, 94404 (US); SABEV, Ogy, San Jose, 95136 (US); MÜLLER, Sven, 99198 Urbich (DE)
(74) Representative: Mathisen & Macara LLP

(57) **Abstract**

A radio frequency system (900) for a radiation therapy machine includes a first portion containing a first gas (906a) at a first pressure, a second portion containing a second gas (906b) at a second pressure, the second gas being different from the first gas, and a component (904) between the first portion and the second portion, the component containing the first gas at the first pressure.

## Description

### TECHNICAL FIELD

The present invention relates to vacuum and/or high pressure radio frequency (RF) systems for medical linear accelerators. Example embodiments relate to radiation support systems including a klystron.

### BACKGROUND

Current medical linear accelerators use sulfur hexafluoride (SF₆) gas to prevent arcing within radio frequency (RF) waveguide structures.

### SUMMARY

One or more example embodiments relate to a radio frequency (RF) system for a medical linear accelerator configured to reduce or prevent arcing without the use of sulfur hexafluoride (SF₆) gas.

One or more example embodiments provide a vacuum-based RF system for a medical linear accelerator.

One or more example embodiments provide an RF system based on a reduced amount of SF₆ gas and a large amount of another gas for a medical linear accelerator.

One or more example embodiments provide a high-pressure gas RF system for a medical linear accelerator.

At least one example embodiment provides a radio frequency system for a radiation therapy machine, the radio frequency system including a first portion containing a gas, a second portion containing the gas, a component between the first portion and the second portion, and a vacuum pump configured to generate a vacuum state inside the component.

The gas may be a non-reactive gas.

The gas may be N₂.

The radio frequency system may further include a regulator configured to maintain a pressure of the gas at 1 atm to 2 atm in the first portion and the second portion.

The component may be a circulator. The circulator may be configured to operate in a vacuum state. A vacuum pump may be connected to the circulator to maintain a vacuum state. Any suitable vacuum pump may be used, for example an ion pump. The vacuum pump may be capable of maintaining a sufficient vacuum state in the circulator. For example, the vacuum pump may maintain a pressure of under about 10⁻⁸ Torr inside the circulator.

The component may include a plurality of openings and the vacuum pump may be configured to generate the vacuum state inside the component through the plurality of openings.

At least one of the first portion or the second portion may include a waveguide. In an embodiment at least one of the first portion or the second portion includes a waveguide and the component includes a plurality of openings, wherein each of the plurality of openings may be smaller than a wavelength of a radio frequency signal of the waveguide. The wave guide may have a rectangular cross-section with a pair of opposing short walls and a pair of opposing long walls. In some embodiments, the vacuum pump is attached to a short wall of the waveguide. A vacuum pump may be attached on a section of a waveguide. The vacuum pump may be attached to the waveguide near a port of the circulator or an RF load of the circulator.

The plurality of openings may be slits. The openings may have a diameter that is smaller than a wavelength of an RF signal of the RF system. For example, the diameter of the openings may be about 1/10 of the size of the RF wavelength. The plurality of openings may be a plurality of slits. A first diameter of an opening in a first direction may be greater than a second diameter of the opening in a second direction perpendicular to the first direction. The first diameter may be smaller than the wavelength of the RF signal of the RF system. The first and second diameters may vary from one opening to another. The first and second diameters may be the same for each of the plurality of openings. The orientation of the plurality of slits may be such that the second diameter aligns with a wall current direction of the section of waveguide. The plurality of openings may have a shape different from slits. For example, the plurality of openings may be a grid of holes with each hole of the grid of holes having the first diameter.

The vacuum pump may be attached to the component with conflat flanges. The vacuum pump may be attached to an attachment on a section of a waveguide, optionally via a tube. The tube for attaching a vacuum pump to a waveguide may comprise any suitable material, including copper or low carbon stainless steel. The attachment may include a plurality of openings.

The vacuum pump may be configured to generate the vacuum state to act as an insulator to reduce arcs within the radio frequency system.

The radio frequency system may further include a first radio frequency window between the first portion and the component, and a second radio frequency window between the second portion and the component.

The radio frequency system may further include a first portion containing a gas at a first pressure, a second portion containing the gas, and a component between the first portion and the second portion, the component containing the gas at a second pressure, the second pressure being different from the first pressure. In embodiments, the radio frequency system comprises a regulator configured to maintain the desired pressure. Embodiments of the invention may comprise a plurality of regulators. A regulator may be connected to a circulator. A regulator may be connected to a waveguide.

The first pressure may be 1 atm to 3 atm and the second pressure may be 3 atm to 5 atm.

The radio frequency system may further include a first radio frequency window between the first portion and the component, and a second radio frequency window between the second portion and the component.

The first pressure may be 3 atm to 5 atm and the second pressure may be 5 atm to 7 atm.

The radio frequency system may further include a first radio frequency window between the first portion and the component, and a second radio frequency window between the second portion and the component, wherein the first radio frequency window and the second radio frequency window are block windows.

The radio frequency system may further include a third portion between the first portion and a rotary joint, the third portion containing the gas at a third pressure, a fourth portion between the second portion and a radio frequency source, the fourth portion containing the gas at the third pressure, a third radio frequency window between the third portion and the first portion, and a fourth radio frequency window between the second portion and the fourth portion. The third and/or fourth RF windows may include thicker ceramics. For example, the ceramic of the third and/or fourth RF windows may be about 20% to 50% thicker than the ceramic of a standard thin disk RF window. For example, the ceramic of the third and/or fourth RF windows and may be about 3mm to about 6mm. The third and/or fourth waveguide may comprise tuning elements. Such tuning elements may include inductive and/or capacitive posts and/or irises near the ceramic. Alternatively, the third and/or fourth RF windows may be a block window, a thick disk window or a Thomson planar window. In embodiments of the invention one or more waveguides is provided by a double ridged waveguide.

The third pressure may be 1 atm to 3 atm.

The component may be a circulator. The circulator may be configured to operate in a vacuum state.

The gas may be a non-reactive gas.

The first pressure may be lower than the second pressure.

At least one example embodiment provides a radio frequency system for a radiation therapy machine, the radio frequency system including a first portion containing a first gas at a first pressure, a second portion containing the first gas, and a component between the first portion and the second portion, the component containing a second gas at a second pressure, the second gas being different from the first gas.

The first pressure may be the same as the second pressure.

The second gas may be SF₆ and the first gas may be a non-reactive gas different from SF₆.

The first gas may be N₂.

The radio frequency system may further include a first radio frequency window between the first portion and the component, and a second radio frequency window between the second portion and the component.

At least one example embodiment provides a radio frequency system for a radiation therapy machine, the radio frequency system including a first portion containing a first gas at a first pressure, a second portion containing a second gas at a second pressure, the second gas being different from the first gas, and a component between the first portion and the second portion, the component containing the first gas at the first pressure.

The first pressure may be the same as the second pressure.

The first gas may be SF₆ and the second gas may be a non-reactive gas different from SF₆.

The second gas may be N₂.

The radio frequency system may further include a rotary joint and an RF source, wherein the component is between the rotary joint and the RF source.

### BRIEF DESCRIPTION OF THE DRAWINGS

Other objects and features of the present invention will become apparent from the following detailed description considered in conjunction with the accompanying drawings.

The drawings, however, are only examples and schematic solely for the purpose of illustration and do not limit the present invention. In the drawings:
FIG. 1 is an example of a conventional radio frequency (RF) system of a radiation therapy machine using SF₆ gas.
FIG. 2 is a comparative example of a RF system of a radiation therapy machine using N₂ gas with a circulator designed to handle N₂ gas at 2 atm pressure..
FIG. 3 is a RF system of a radiation therapy machine according to example embodiments.
FIG. 4A is a top view illustrating a configuration of the vacuum pump attached to the circulator, according to example embodiments.
FIG. 4B is an example of an attachment according to example embodiments.
FIG. 5 is a RF system of a radiation therapy machine according to example embodiments using N₂ gas with a circulator designed to handle N₂ gas at 4 atm pressure.
FIG. 6 is a RF system of a radiation therapy machine according to example embodiments using N₂ gas with a circulator designed to handle N₂ gas at 6 atm pressure.
FIG. 7 is a RF system of a radiation therapy machine according to example embodiments.
FIGS. 8A and 8B are examples of a side view and a top view of a double ridged waveguide.
FIG. 9 is a RF system of a radiation therapy machine according to example embodiments using N₂ gas in some areas and SF₆ gas in other areas.
FIG. 10 is an attachment according to example embodiments.
FIG. 11 is an example of Paschen Curves of some example gases.
FIG. 12 is an example of Paschen Curves quantitatively comparing SF6 and N2 gases.
FIG. 13 is a RF system according to example embodiments.

### DETAILED DESCRIPTION

Various example embodiments will now be described more fully with reference to the accompanying drawings in which only some example embodiments are shown. Specific structural and functional details disclosed herein are merely representative for purposes of describing example embodiments. Example embodiments, however, may be embodied in various different forms, and should not be construed as being limited to only the illustrated embodiments. Rather, the illustrated embodiments are provided as examples so that this disclosure will be thorough and complete, and will fully convey the concepts of this disclosure to those skilled in the art. Accordingly, known processes, elements, and techniques, may not be described with respect to some example embodiments. Unless otherwise noted, like reference characters denote like elements throughout the attached drawings and written description, and thus descriptions will not be repeated. The present invention, however, may be embodied in many alternate forms and should not be construed as limited to only the example embodiments set forth herein.

Detailed illustrative embodiments are disclosed herein. However, specific structural and functional details disclosed herein are merely representative for purposes of describing example embodiments. The example embodiments may, however, be embodied in many alternate forms and should not be construed as limited to only the embodiments set forth herein.

When the words "about" and "substantially" are used in this application in connection with a numerical value, it is intended that the associated numerical value include a tolerance of ±10% around the stated numerical value, unless otherwise explicitly defined. Further, regardless of whether numerical values are modified as "about" or "substantially," it will be understood that these values should be construed as including a of ±10% around the stated numerical value. When the word "about" is used in this application in connection with a numerical value of a pressure, it is intended that the associated numerical value include a tolerance of ±1. For example, a waveguide configured to operate at about 2 atm could be configured to operate between 1 atm to 3 atm.

Independent of the grammatical term usage, individuals with male, female or other gender identities are included within the term.

Accelerated electrons from linear accelerators (LINACs) may subsequently be used to generate the x-rays of a specific energy, for example for treatment of a patient. The LINACs may be a type of Vacuum Electron Device (VED), which uses relatively high power microwave signals to operate. These microwave signals are produced by a relatively high power microwave source, such as a klystron and/or a magnetron.

The microwave/radio frequency (RF) power may be conducted from a source to the LINAC via a network of waveguides. While the VEDs are under ultra-high vacuum, the waveguides may be pressurized with a dielectric gas.

Electric fields inside the hollow waveguides of a RF system, such as those mentioned above, may be relatively high. The dielectric gas may be used to reduce and/or prevent the likelihood of and/or impact from electrical breakdown in the waveguides. While traveling from the microwave source to a LINAC, these high-power microwaves may pass through various components, such as circulators, flexible waveguides, rotary joints, couplers, water loads, phase wands, RF windows, etc.

Some components, for example circulators, may produce undesired arcing. To reduce or prevent the arcing, some radiation therapy machines employ sulfur hexafluoride (SF₆) gas to act as an insulator to suppress arcing in a circulator.

Some radiation therapy machines have reduced SF₆ gas emissions into the open atmosphere by using SF₆ recovery equipment (including pumps) when manufacturing, servicing, and/or repairing the machines. However, the SF₆ recovery process does not address gradual SF₆ leakage that may occur under normal operation. In some cases, the amount of SF₆ that leaks out can significantly exceed the amount of SF₆ recovered during servicing.

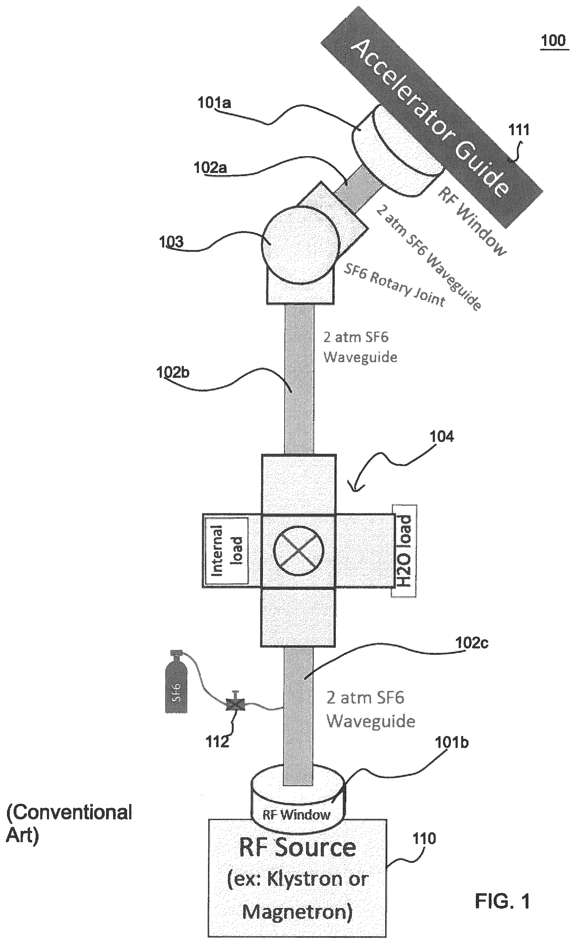

FIG. 1 is an example of a RF system of a radiation therapy machine using SF₆ gas.

Referring to FIG. 1, RF system 100 may include first and second RF windows 101a and 101b, first to third waveguides 102a to 102c, a rotary joint 103, a circulator 104, a RF source 110, and/or an accelerator guide 111. The waveguides 102a to 102c may be configured to operate at an internal gas dielectric pressure of about 2 atm. As used herein the waveguides 102a to 102c may be referred to as waveguides, waveguide portions, or portions. As used herein, the circulator 104 may be referred to as a component.

The RF source 110 may be or may include a source of RF energy, such as electromagnetic energy in the radio wave and/or microwave spectrum. For example, the RF source 110 may be or may include at least one of a magnetron or a klystron. The RF source 110 may generate electromagnetic radiation, and the electromagnetic radiation may propagate through the RF system 100 to the accelerator guide 111.

The RF system 100 may include the first RF window 101a coupled between the accelerator guide 111 and the first waveguide 102a, a rotary joint 103 coupled between the first waveguide 102a and the second waveguide 102b, the circulator 104 coupled between the second waveguide 102b and the third waveguide 102c, and the third waveguide 102c coupled between the circulator 104 and the second RF window 101b, and the RF source 110 coupled to the second RF window 101b. The RF windows may be pillbox type RF windows, thin disk type RF windows, etc.

The circulator 104 acts as a kind of one-way valve for microwave energy to protect the RF source 110 by diverting and absorbing any microwave energy that is reflected back from the accelerator guide 111.

For example, at a start of a beam-on operation, for the first pulses (e.g., the first tens of pulses) non-resonant RF pulses may be reflected backwards from the accelerator guide 111. For example, the non-resonant RF pulses may be reflected backwards in the first RF window 101a, the first waveguide 102a, the rotary joint 103, and/or the second waveguide 102b. The reflected portion of the pulse adds to the rest of the incoming pulse to form an electric field that can be twice as strong as normal. Areas of the RF system 100 with double strength electric fields have a higher probability of experiencing arcing. The circulator 104 may reduce or prevent reflected waves from reaching and damaging the RF source 110.

The accelerator guide 111 and the RF source 110 may be maintained in a vacuum state. For example, no pressurized gas may be maintained in the accelerator guide 111 and/or the RF source 110. For example, a vacuum state in the accelerator guide 111 and the RF source 110 may be maintained at a pressure under about 10⁻⁸ Torr.

In the RF system 100, as shown in FIG. 1, SF₆ at a pressure of about 2 atm may be maintained in the waveguides 102. For example, a regulator 112 may be configured to maintain a pressure in the waveguides 102 and the circulator 104 at about 2 atm. The circulator 104 may be configured to operate with SF₆ at the pressure of about 2 atm to act as an insulator to prevent arcing inside the circulator 104. However, SF₆ is a highly environmentally damaging greenhouse gas. Accordingly, a cost-effective alternative to using SF₆ gas to prevent arcing inside the circulator is desired.

A possible alternative to using a circulator configured to operate using the SF₆ gas is a circulator configured to operate using another gas. For example, nitrogen (N₂).

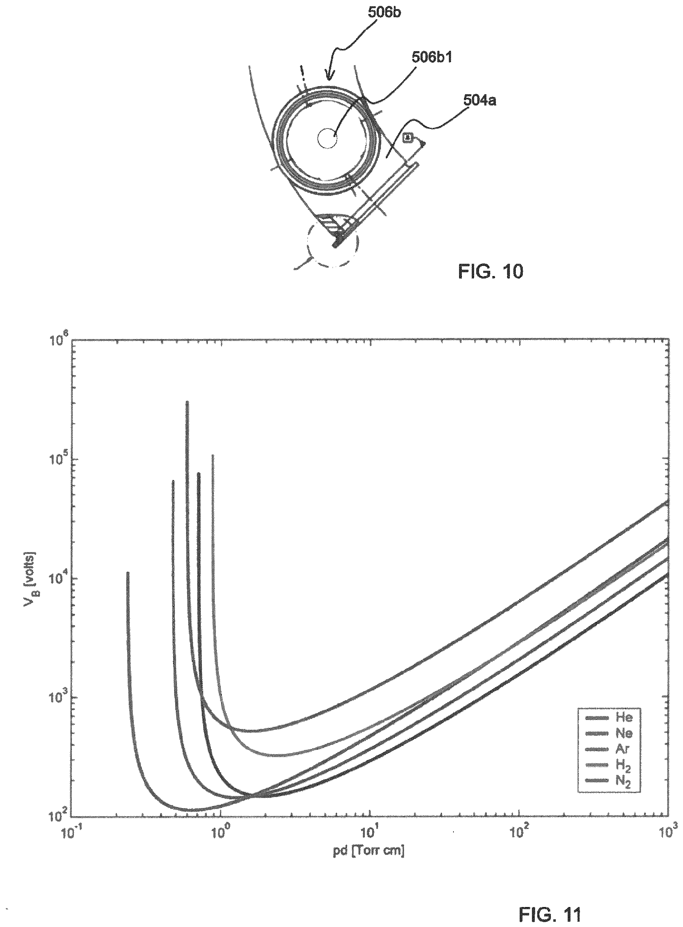

FIG. 11 is an example of Paschen Curves of some example gases.

A Paschen Curve of a given gas indicates a breakdown voltage of the gas depending on a pressure. Referring to FIG. 11, example Paschen curves are shown for helium (He), neon (Ne), argon (Ar) hydrogen (H₂) and nitrogen (N₂). The vertical axis indicates a breakdown voltage V_{B} and the horizontal axis indicates a combination of pressure and electrode gap distance (Torr cm). As may be appreciated from the example Paschen Curves shown in FIG. 11, at high gas pressure, a high breakdown voltage, V_{B}, is required to cause an arc (right-hand side of FIG. 11). As the gas pressure drops, an arc can be created with a lower voltage V_{B} (center of FIG. 11), until the gas pressure is so low that it is mostly a vacuum, in which case the vacuum acts as an insulator to stop arcs (left-hand side of FIG. 11).

The scale of the horizontal axis of the Paschen Curves may depend on the geometry of a system containing the respective gases.

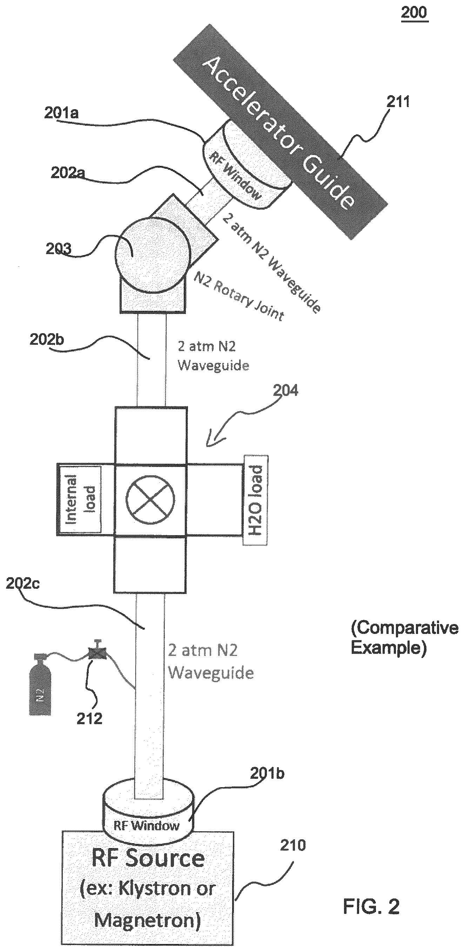

FIG. 2 is a comparative example of a RF system of a radiation therapy machine.

Referring to FIG. 2, a RF system 200 may include first and second RF windows 201a and 201b, first to third waveguides 202a to 202c, a rotary joint 203, a circulator 204, a RF source 210, an accelerator guide 211, and/or a regulator 212. As used herein the waveguides 202a to 202c may be referred to as waveguides, waveguide portions, or portions. As used herein, the circulator 204 may be referred to as a component. Descriptions of FIG. 2 similar to FIG. 1 will be omitted and the differences will be mainly described.

Different from the RF system 100, the RF system 200 may be configured to operate using N₂ gas instead of SF₆ gas. For example, the first to third waveguides 202a to 202c and the circulator 204 may include the N₂ gas at the pressure of about 2 atm.

The circulator 204 may be configured to operate with the N₂ gas at the pressure of about 2 atm to act as an insulator to prevent arcing inside the circulator 204. However, it is noted that the circulator of the RF system 100 (e.g., the circulator 104) may not operate by simply replacing the SF₆ gas with the N₂ gas. It is estimated that a circulator configured to operate with the N₂ gas, such as the circulator 204, may be about 1.5x to 2x the volume of the circulator 104 and may be significantly more expensive to produce. Accordingly, a more compact and cost-effective alternative to using SF₆ gas to prevent arcing inside the circulator is desired.

According to some example embodiments, and as discussed above with reference to FIG. 11, a vacuum state may be maintained in a circulator to act as an insulator to suppress (e.g., reduce and/or prevent) arcing in the circulator.

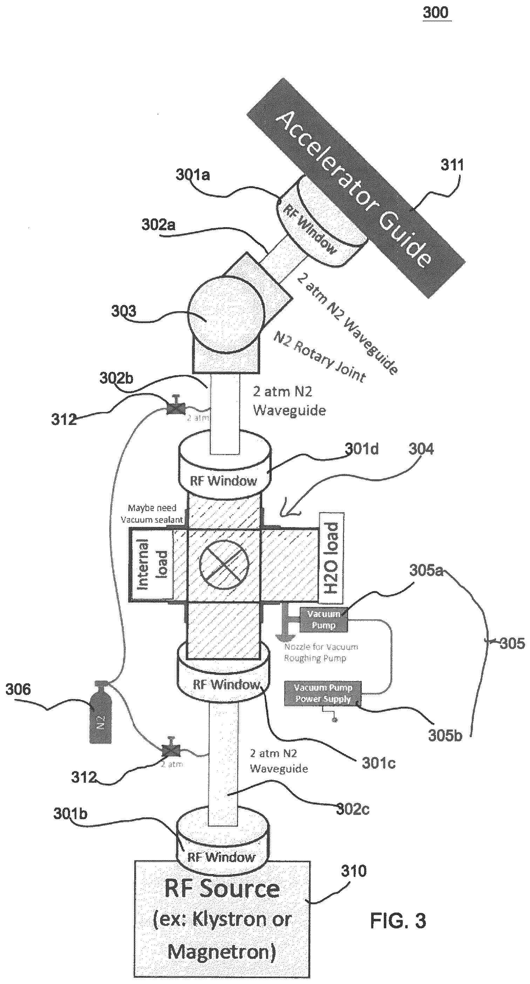

FIG. 3 is a RF system of a radiation therapy machine according to example embodiments.

Referring to FIG. 3, a RF system 300 according to example embodiments includes first to fourth RF windows 301a to 301d, first to third waveguides 302a to 302c, a rotary joint 303, a circulator 304, a vacuum pump 305, a non-reactive gas supply 306, regulators 312, a RF source 310, and/or an accelerator guide 311. As used herein the waveguides 302a to 302c may be referred to as waveguides, waveguide portions, or portions. As used herein, the circulator 304 may be referred to as a component. Descriptions of FIG. 3 similar to FIGS. 1 and 2 will be omitted and the differences will be mainly described.

More specifically, the RF system 300 includes first RF window 301a coupled between accelerator guide 311 and first waveguide 302a, rotary joint 303 coupled between first waveguide 302a and second waveguide 302b, fourth RF window 301d coupled between second waveguide 302b and circulator 304, third RF window 301c coupled between circulator 304 and third waveguide 302c, and second RF window 301b coupled between third waveguide 302c and RF source 310. The components may be coupled together in a conventional manner.

First to fourth RF windows 301a to 301d may be, for example, medical grade thin wall RF windows. Medical grade thin wall RF windows have proven effective at working for years transmitting 5.5 MW RF pulses across a gas pressure differential of 2 atm.

Different from the comparative example shown in FIG. 2, the circulator 304 may be the same or similar to the conventional circulator 104, except that the circulator 304 is configured to operate in a vacuum state. For example, a vacuum pump 305 may be attached to the circulator 304 to maintain the vacuum state. Additionally, the circulator 304 may include additional vacuum sealant (not shown) to maintain the vacuum state.

The vacuum pump 305 includes a vacuum pump 305a and a vacuum pump power supply 305b. The vacuum pump 305a may be an ion pump. However, example embodiments are not limited thereto and the vacuum pump 305a may be any vacuum pump capable of maintaining a sufficient (e.g., ultra high) vacuum state in the circulator 304. For example, the vacuum pump may maintain a pressure of under about 10⁻⁸ Torr inside the circulator 304. However, example embodiments are not limited thereto. For example, Paschen Curves of gases, as discussed above with regard to FIG. 11, show high insulation at high vacuum but the units of the horizontal scale (e.g., pressure) depend on geometrical details (e.g., geometrical details of the circulator 304), Accordingly the pressure maintained inside the circulator 304 by the vacuum pump 305 may vary according to geometrical details of the circulator 304. The vacuum pump power supply 305b may supply a high voltage to the vacuum pump 305a.

According to some example embodiments, a one-time process may be performed to generate the vacuum state inside the circulator 304. For example, a roughing pump and then a turbo pump may be attached via nozzles to the circulator 304 to generate a strong enough vacuum state for the ion pump to function. For example, the roughing pump and/or the turbo pump may be used to generate the vacuum state in the circulator 304 during manufacture and may be removed from the circulator 304 prior to shipment of the circulator 304. The vacuum pump 305a may be left attached to the circulator 304 when the circulator is shipped. During shipping, the vacuum pump 305a may be powered by a battery, however example embodiments are not limited thereto and the vacuum state may be maintained without constant power to the vacuum pump 305a such that the desired pressure can be restored after shipping by running the vacuum pump 305. For example, the vacuum pump 305a may be permanently affixed to the circulator 304.

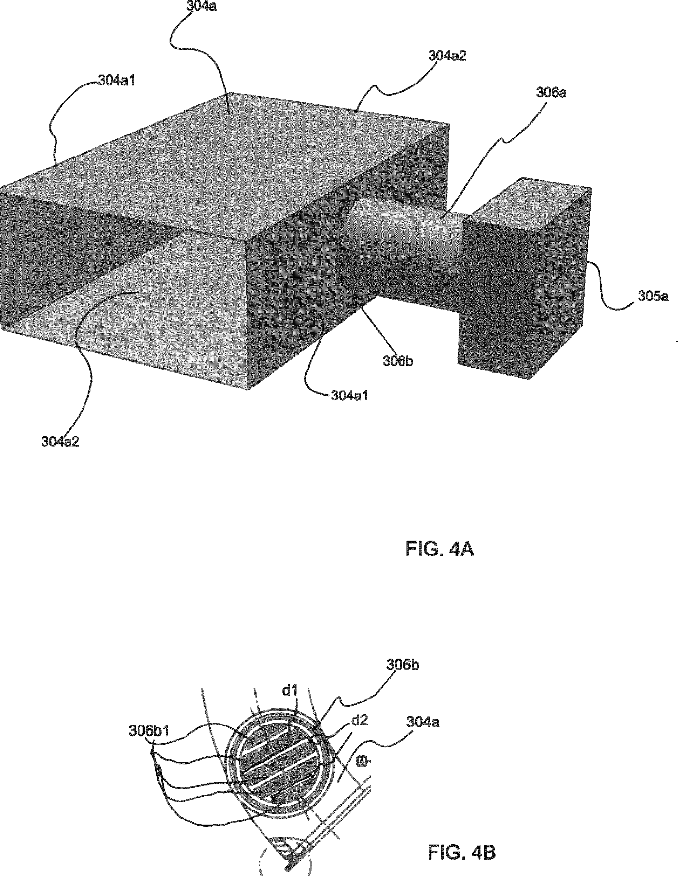

FIG. 4A is a top view illustrating a configuration of the vacuum pump attached to the circulator, according to example embodiments.

Referring to FIG. 4A, a waveguide 304a included in the circulator 304 may have a rectangular cross-section. For example, the waveguide 304a may include short walls 304a1 and long walls 304a2. Attaching the pump on the short walls 304a1 only has the potential to disrupt RF currents which can be corrected for by choosing the aperture carefully. The electric fields along the short walls 304a1 are essentially zero, while electric fields along the long walls 304a2 reach a maximum near the middle of the walls and would be much more likely to initiate an arc.

As shown in FIG. 4A, the vacuum pump 305a may be attached to a section of a short wall 304a1 of the waveguide 304a included in the circulator 304.

The section of waveguide 304a may be a section of waveguide not part of the core of the circulator but near a port of the circulator or an RF load of the circulator. For example, the circulator 304 may be a 3 or 4 port circulator, with the ports referring to openings where the RF waves enter and/or exit the circulator 304. RF waves only enter and exit in one specific direction each of the ports of the circulator 304. RF waves entering one port (e.g., input) must exit from a specific other port (e.g., output) based on which input is used. In the forward direction RF waves enter and pass through the circulator 304, while reflected power is diverted to a different output where a water load absorbs the RF of the reflected power, dissipating it. A load is needed at a specific port to absorb RF coming back from the LINAC so it does not go back to the RF source 310. Accordingly, placement of the vacuum pump attachment near a port of the circulator 304 or an RF load of the circulator 304 may result in a reduced (e.g., minimal) risk of disrupting an RF wave as it interacts with circulator ferrites near a core of the circulator 304 during operation.

The vacuum pump 305a may be attached (e.g., coupled) to an attachment 306b on the section of waveguide 304a via a tube 306a. The tube 306a may be, for example, a copper tube. However, example embodiments are not limited thereto and the tube 306a may be any suitable material such as low carbon stainless steel.

FIG. 4B is an example of an attachment according to example embodiments.

Referring to FIG. 4B, the attachment 306b may include a plurality of openings 306b1. The openings 306b1 may have a diameter that is smaller than a wavelength of an RF signal of the RF system 300. For example, a diameter of the openings 306b1 may be a fraction of the size of the RF wavelength. For example, the diameter of the openings 306b1 may be about 1/10 of the size of the RF wavelength. The plurality of openings 306b1 may be numerous enough to provide enough flow for good vacuum conductance. Therefore, the openings 306b1 may allow the vacuum pump 305a to maintain the vacuum state inside the circulator while reducing or preventing RF from leaking from the circulator 304.

As shown in FIG. 4B, the plurality of openings 306b1 may be a plurality of slits. For example, a diameter d1 of an opening 306b1 in a first direction may be greater than a diameter d2 of the opening 306b1 in a second direction perpendicular to the first direction. The diameter d1 may be smaller than the wavelength of the RF signal of the RF system 300. However, the diameter d2 may not be significantly larger due to its parallel orientation with the RF wall currents which does not disrupt the RF wave as much as d1 would. For example, as shown in FIG. 4B, the diameter d2 may vary for the plurality of slits 306b1. The orientation of the plurality of slits 306b1 may line up with a wall current direction of the section of waveguide 304 (e.g., the plurality of slits 306b1 may be oriented parallel to the electric field in the waveguide). For example, the diameter d2 may be in a direction of the wall current. However, example embodiments are not limited thereto and the plurality of openings 306b1 may be some other shape different from slits. For example, the plurality of openings 306b1 may be a grid of holes with each hole of the grid of holes having the diameter d1. A grid of holes may be more symmetric than the slits, but have worse gas conductance.

The tube 306a may be attached to the vacuum pump 305a and/or to the attachment 306b in a manner to create a very good seal such that the net gas inflow does not exceed what the pump 305a can remove. For example, the tube 306a may be attached to the vacuum pump 305a and/or to the attachment 306b via brazed conflat flanges. However, example embodiments are not limited thereto and any suitable vacuum connection device may be used.

Returning to FIG. 3, different from FIGS. 1 and 2, the RF system 300 may include the third RF window 301c coupled between the circulator 304 and the third waveguide 302c and the fourth RF window 301d coupled between the circulator 304 and the second waveguide 302b. The waveguides 302a to 302c may be configured to operate at a pressure of about 2 atm. Accordingly, regulators 312 may be configured to maintain the pressure in the waveguides at about 2 atm.

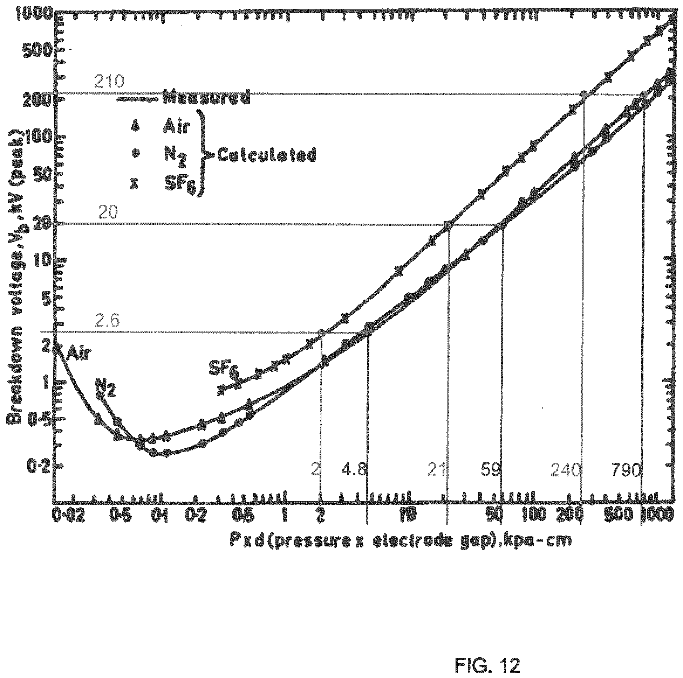

The non-reactive gas supply 306 may supply a non-reactive gas. For example, the non-reactive gas supply 306 may supply N₂ gas. However, example embodiments are not limited thereto and any non-reactive gas may be used. However, as discussed above with reference to FIG. 12, a different non-reactive gas may be maintained at different pressures than those recited herein with regard to the examples using N₂ gas. For ease of discussion the non-reactive gas may be referred to as N₂ gas herein. However, it is understood that the N₂ may be replaced with another non-reactive gas other than SF₆.

FIG. 12 is an example of Paschen Curves of some example gases quantitatively comparing SF₆ and N₂ gases.

Referring to FIG. 12, example Paschen Curves are shown for N₂, SF₆, and air. The vertical axis indicates a breakdown voltage V_{B} and the horizontal axis indicates a combination of pressure and distance between electrodes, Torr cm.

As may be appreciated by the Paschen Curves shown in FIG. 12, to suppress arcing in the circulator 304 using N₂ gas, the N₂ gas needs to be maintained at a pressure between 2.3x-3.5x the pressure of SF₆ gas. For example, to replace SF₆ gas in the circulator 104 configured for SF₆ at 2 atm, circulator 304 needs to maintain a pressure of the N₂ gas at about 5 atm - 7 atm.

As shown in FIG. 3, the RF system 300 may include at least one regulator 312 attached to the waveguides 302b and/or 302a and at least one regulator 312 attached to the waveguide 302c. For example, the RF system 300 may include at least one regulator on either side of the circulator 304 configured to maintain the pressure of the N₂ gas at about 2 atm in the RF system between the first RF window 301a and the second RF window 301b, except for in the circulator 304 which is maintained in a vacuum state. However, example embodiments are not limited thereto. For example, the at least one regulator 312 may be configured to maintain the pressure of the N₂ gas in the RF system 300 from the third RF window 301c to the second RF window 301b at about 2 atm, and to maintain the pressure of the N₂ gas from the first RF window 301a to the fourth RF window 301d at a higher pressure (e.g., about 3 atm to about 4 atm) to account for double strength electric fields from reflected RF pulses that may be present in this section of the RF system 300.

Accordingly, the RF system 300 may reduce and/or prevent arcing in the circulator 304 without the use of SF₆. Because the RF system 300 uses existing circulators (e.g., circulator 104) with an added vacuum, rather than a redesigned circulator (e.g., circulator 204) a cost of the RF system 300 may be relatively low and an increase in volume of the RF system 300 may be relatively low.

Some example embodiments provide alternative RF systems employing a non-reactive gas other than SF₆ and without maintaining a vacuum state in the circulator.

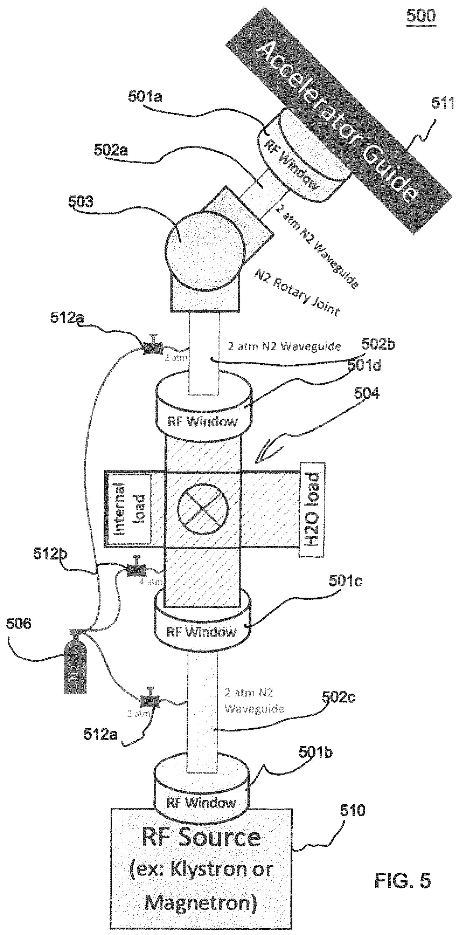

FIG. 5 is a RF system of a radiation therapy machine according to example embodiments.

Referring to FIG. 5, a RF system 500 according to example embodiments may include first to fourth RF windows 501a to 501d, first to third waveguides 502a to 502c, a rotary joint 503, a circulator 504, a non-reactive gas supply 506, first and second regulators 512a and 512b, a RF source 510, and/or an accelerator guide 511. As used herein the waveguides 502a to 502c may be referred to as waveguides, waveguide portions, or portions. As used herein, the circulator 504 may be referred to as a component. Descriptions of FIG. 5 similar to FIGS. 1-3 will be omitted and the differences will be mainly described.

More specifically, the RF system 500 includes first RF window 501a coupled between the accelerator guide 511 and the first waveguide 502a, the rotary joint 503 coupled between the first waveguide 502a and the second waveguide 502b, the fourth RF window 501d coupled between the second waveguide 502b and the circulator 504, the third RF window 501c coupled between the circulator 504 and the third waveguide 502c, and the second RF window 501b coupled between the third waveguide 502c and the RF source 510.

As discussed above, it is not possible to merely replace the SF₆ gas in a circulator designed for SF₆ gas (e.g., circulator 104) with another non-reactive gas (e.g., N₂). Rather, as discussed with reference to FIG. 2, in order to use N₂, a circulator designed for N₂ at about 2 atm to reduce or prevent arcing inside the circulator may need to be designed. However, the RF system 500, different from the RF system 200, may use a different non-reactive gas (e.g., N₂) at a higher pressure (e.g., about 4 atm) in a current circulator (e.g., circulator 104) to create an insulator to reduce or prevent arcing inside the circulator 504 without designing a new circulator.

For example, different from the comparative example described in FIG. 2, the RF system 500 includes the third RF window 501c coupled between the circulator 504 and the third waveguide 502c and the fourth RF window 501d coupled between the circulator 504 and the second waveguide 502b. The third and fourth RF windows 501c and 501d may be stand-alone windows with a small section of waveguide and RF flanges that bolt on to flanges of the circulator 504.

The waveguides 502a to 502c may be configured to operate at a pressure of about 2 atm. Accordingly, the first regulators 512a may be configured to maintain the pressure in the waveguides at about 2 atm. For example, the RF system 500 may include at least one first regulator 512a attached to the waveguides 502b and/or 502a and at least one first regulator 512a attached to the waveguide 502c. For example, the RF system 500 may include at least one regulator on either side of the circulator 504 configured to maintain the pressure of the N₂ gas at about 2 atm in the RF system 500 between the first RF window 501a and the second RF window 501b, except for in the circulator 504 which is maintained at a higher pressure. However, example embodiments are not limited thereto. For example, the at least one regulator 512 may be configured to maintain the pressure of the N₂ gas in the RF system 500 from the third RF window 501c to the second RF window 501b at about 2 atm, and to maintain the pressure of the N₂ gas from the first RF window 501a to the fourth RF window 501d at a higher pressure (e.g., about 3 atm to about 4 atm) to account for double strength electric fields from reflected RF pulses that may be present in this section of the RF system 500.

For example, the circulator 504 may be configured to operate with the non-reactive gas at a pressure of about 4 atm to act as an insulator to reduce and/or prevent arcing in the circulator 504. Accordingly, the regulator 512b may be configured to maintain the pressure in the circulator 504 at about 4 atm.

FIG. 10 is an attachment according to example embodiments.

Referring to FIGS. 5 and 10, the second regulator 512b may connect to an attachment 506b on side of a waveguide 504a of the circulator 504 via a small opening 506b1 about .1" to about .2" in diameter. This is enough to let pressure in and not allow RF out. Different from the attachment 506b, multiple holes or slits are not needed to maintain gas pressure in the circulator 504, as N₂ can easily fill through one hole about 1" to about .2" in diameter.

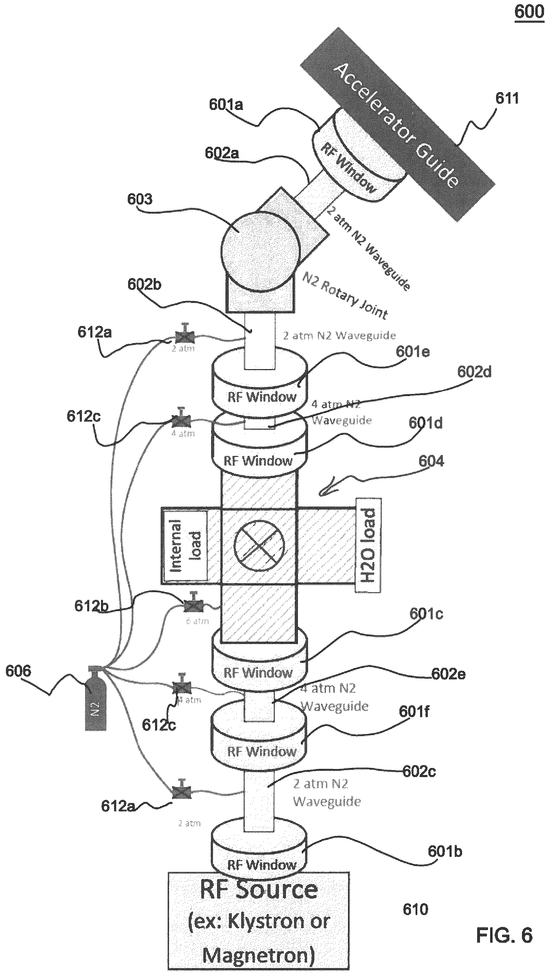

FIG. 6 is a RF system of a radiation therapy machine according to example embodiments.

Referring to FIG. 6, a RF system 600 according to example embodiments may include first to sixth RF windows 601a to 601f, first to fifth waveguides 602a to 602e, a rotary joint 603, a circulator 604, a non-reactive gas supply 606, first to third regulators 612a to 612c, a RF source 610, and/or an accelerator guide 611. As used herein the waveguides 602a to 602e may be referred to as waveguides, waveguide portions, or portions. As used herein, the circulator 604 may be referred to as a component. Descriptions of FIG. 6 similar to FIGS. 1-3 and 5 will be omitted and the differences will be mainly described.

Different from FIG. 5, the circulator 604 may be configured to use a non-reactive gas other than SF₆ (e.g., N₂) at about 6 atm in current circulator 604 to create an insulator to reduce or prevent arcing inside the circulator 604. Similar to the circulator 504 discussed with reference to FIG. 5, the circulator 604 may be a current circulator (e.g., circulator 104).

A given RF window may be limited in how much pressure difference it can withstand before cracking from mechanical stress. For example, an RF window may last for years sustaining a pressure difference of 2 atm, but it may not be known how long such an RF window could sustain a 3 atm pressure difference. To reduce the pressure on the RF windows, RF system 600 employs a staged approach to ensure each RF window sees no more than a 2 atm pressure difference from one side of a ceramic of the waveguide to the other side of the ceramic.

Specifically, different from FIG. 5, the RF system 600 may include the fifth and sixth RF windows 601e and 601f and the fourth and fifth waveguides 602d and 602e. The fourth and fifth waveguides may be configured to operate with a non-reactive gas at 4 atm.

Specifically, referring to FIG. 6, the RF system 600 includes the first RF window 601a coupled between the accelerator guide 611 and the first waveguide 602a, the rotary joint 603 coupled between the first waveguide 602a and the second waveguide 602b, the fifth RF window 601e coupled between the second waveguide 602b and the fourth waveguide 602d, the fourth RF window 601d coupled between the fourth waveguide 602d and the circulator 604, the third RF window 601c coupled between the circulator 604 and the fifth waveguide 602e, the sixth RF window 601f coupled between the fifth waveguide 602e and the third waveguide 602c, and the fourth RF window 601b coupled between the third waveguide 602c and the RF source 610.

A section of the RF system 600 between the first RF window 601a and the fifth RF window 601e and a section of the RF system 600 between the second RF window 601b and the sixth RF window 601f may contain the non-reactive gas at about 2 atm. For example, at least one first regulator 612a may be connected to at least one of the first waveguide 602a and/or the second waveguide 602b and at least one first regulator 612a may be connected to the third waveguide 602c. The first regulators 612a may be configured to maintain the non-reactive gas at about 2 atm.

A section of the RF system 600 between the fifth RF window 601e and the fourth RF window 601d and a section of the RF system 600 between the third RF window 601c and the sixth RF window 601f may contain the non-reactive gas at about 4 atm. For example, at least one third regulator 612c may be connected to the fourth waveguide 602d and at least one third regulator 612c may be connected to the fifth waveguide 602e. The third regulators 612c may be configured to maintain the non-reactive gas at about 4 atm.

A second regulator 612b may be connected to the circulator 604. The second regulator 612b may maintain the non-reactive gas in the circulator at about 6 atm to act as an insulator to suppress arcing in the circulator 604.

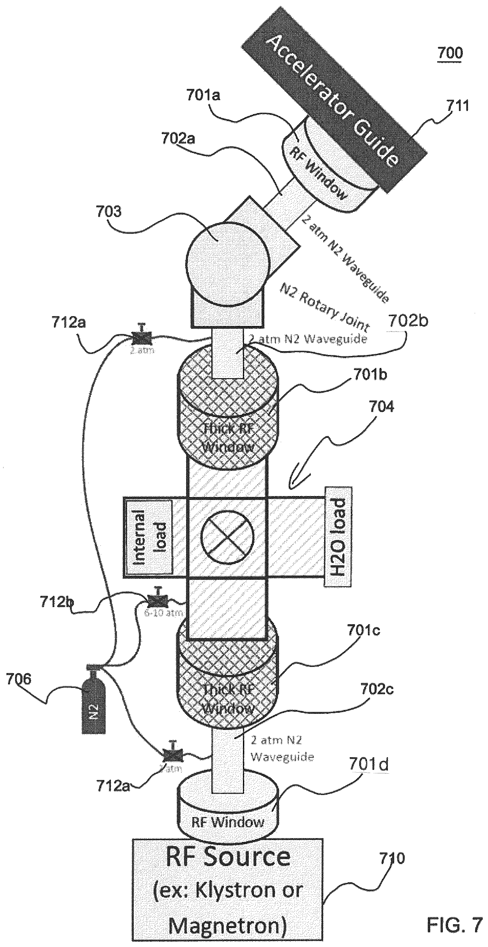

FIG. 7 is a RF system of a radiation therapy machine according to example embodiments.

Referring to FIG. 7, a RF system 700 according to example embodiments may include first to fourth RF windows 701a to 701d, first to third waveguides 702a to 702c, a rotary joint 703, a circulator 704, a non-reactive gas supply 706, first and second regulators 712a and 712b, a RF source 710, and/or an accelerator guide 711. As used herein the waveguides 702a to 702c may be referred to as waveguides, waveguide portions, or portions. As used herein, the circulator 704 may be referred to as a component. Descriptions of FIG. 7 similar to FIGS. 1-3 and 5-6 will be omitted and the differences will be mainly described.

More specifically, the RF system 700 includes the first RF window 701a coupled between the accelerator guide 711 and the first waveguide 702a, the rotary joint 703 coupled between the first waveguide 702a and the second waveguide 702b, the fourth RF window 701d coupled between the second waveguide 702b and the circulator 704, the third RF window 701c coupled between the circulator 704 and the third waveguide 702c, and the second RF window 701b coupled between the third waveguide 702c and the RF source 710.

First regulators 712a are configured to maintain a pressure of the non-reactive gas in the waveguides 702a to 702c at about 2 atm. Second regulator 712b is configured to maintain a pressure of the non-reactive gas in the circulator 704 at a pressure of about 6 atm to about 10 atm. For example, 8 atm. However, example embodiments are not limited thereto. For example, the first regulators 712a may maintain the pressure of the non-reactive gas from the first RF window 701a to the second RF window 701b at a higher pressure (e.g., about 3 atm to about 4 atm) to account for double strength electric fields from reflected RF pulses that may be present in this section of the RF system 700.

Different from FIG. 6, instead of employing a staged approach to ensure each RF window sees no more than a 2 atm pressure difference from one side of the ceramic to the other side, the third and fourth RF windows 701c and 701d may be configured to withstand a greater atm pressure difference.

For example, the third and fourth RF windows 701c and/or 701d may include thicker ceramic than standard thin disk RF windows. Making the ceramic even a little thicker may greatly enhance the strength of the RF window.

The third and/or fourth RF windows 701c and 701d may include thicker ceramics. For example, the ceramic of the third and/or fourth RF windows 701c and 701d may be about 20% to 50% thicker than the ceramic of a standard thin disk RF window. The thicker ceramic may be accomplished by changing the waveguide shape of the RF window near the window ceramic, and/or by adding tuning elements. Such tuning elements may include inductive and/or capacitive posts and/or irises near the ceramic. For example, the ceramic of the third and/or fourth RF windows 701c and 701d may be about 3mm to about 6mm.

Alternatively, the third and/or fourth RF windows 701c and 701d may be a block window or a thick disk window. These windows have a rectangular ceramic and are not known as pillboxes. Different from standard pillbox or thin disk windows, block windows and thick disk windows do not include a circular waveguide section around a circular ceramic. Block windows and thick disk have a narrower bandwidth than pillbox windows and can withstand greater pressure differences. They are called thick disk or block windows because the ceramic is designed to be half the RF wavelength in thickness. For example, the ceramic of the third and/or fourth RF windows 701c and 701d may be about 3 inches for our wavelength. This special property allows RF reflections to internally cancel out and allows all the power to transmit unimpeded.

Alternatively, the third and/or fourth RF windows 701c and 701d may be a Thomson planar window. Thomson planar windows have ceramics smaller in diameter than the waveguide dimensions. The smaller diameter means less surface area exposed to the high pressure, and a higher yield point. To match the RF through such a window clever tuning is needed. This includes tuning elements mentioned above, or alternatively using a double ridged waveguide.

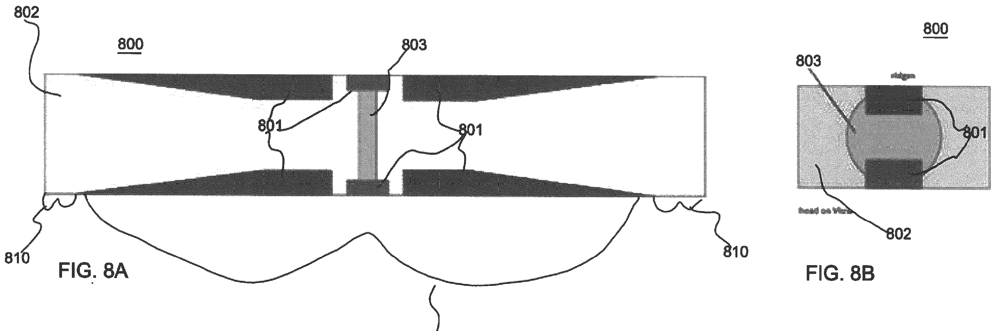

FIGS. 8A and 8B are examples of a side view and a top view of a double ridged waveguide.

Referring to FIGS. 8A and 8B, a double ridged waveguide 800 (e.g., a Thomson planar window) according to example embodiments includes ridges 801, walls 802, and a ceramic 803.

The double ridged waveguide 800 includes an RF window (e.g., the ceramic 803) in the middle. The ceramic 803 is smaller in diameter than the waveguide 800, thus more robust to higher pressure without cracking. A double ridged transition may serve to match the RF through the ceramic 803, which would otherwise have higher reflections.

As shown in FIG. 8A, the double ridged waveguide 800 transitions from a rectangular portion 810 to a double ridged portion 811, then back to a rectangular portion 810 due to a shape of the ridges 801. The ridges 801 may be formed of a metal. For example, the ridges 801 may be formed of a same metal as the walls 802. The ridges 801 may be molded (e.g., machined) into the walls 802 of the double ridged waveguide 800.

The ridges 801 focus the majority of the RF fields through the ceramic. For example, a majority of an RF signal may be focused between the ridges 802 and through the ceramic 803 without reflecting off of the metal (e.g., the walls 802 and/or ridges 801) supporting the ceramic.

Instead of eliminating the use of SF₆ entirely through either a vacuum state or using a different non-reactive gas in the circulator, some example embodiments may use SF₆ to act as an insulator to reduce and/or prevent arcing inside the circulator while greatly reducing an amount of SF₆ used in an RF system.

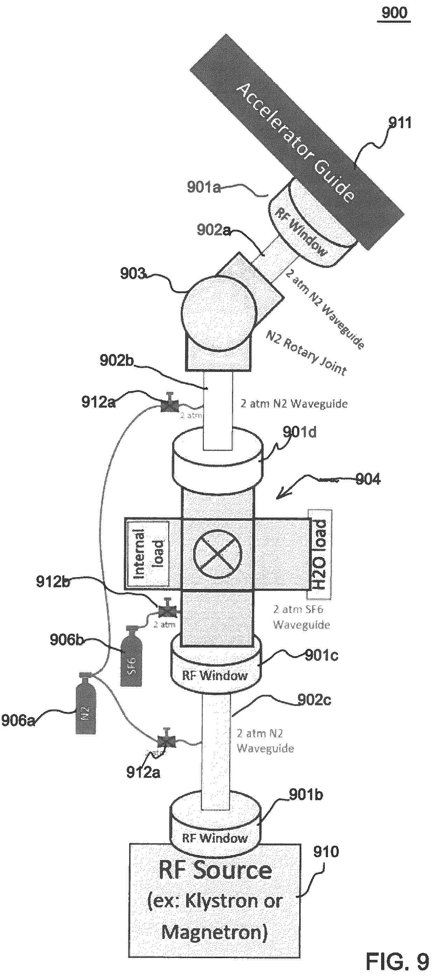

FIG. 9 is a RF system of a radiation therapy machine according to example embodiments.

Referring to FIG. 9, a RF system 900 according to example embodiments may include first to fourth RF windows 901a to 901d, first to third waveguides 902a to 902c, a rotary joint 903, a circulator 904, first and second non-reactive gas supplies 906a and 906b, first and second regulators 912a and 912b, a RF source 910, and/or an accelerator guide 911. As used herein the waveguides 902a to 902c may be referred to as waveguides, waveguide portions, or portions. As used herein, the circulator 904 may be referred to as a component. Descriptions of FIG. 9 similar to FIGS. 1-3 and 5-7 will be omitted and the differences will be mainly described.

More specifically, the RF system 900 includes the first RF window 901a coupled between the accelerator guide 911 and the first waveguide 902a, the rotary joint 903 coupled between the first waveguide 902a and the second waveguide 902b, the fourth RF window 901d coupled between the second waveguide 902b and the circulator 904, the third RF window 901c coupled between the circulator 904 and the third waveguide 902c, and the second RF window 901b coupled between the third waveguide 902c and the RF source 910.

The circulator 904 may be an existing circulator (e.g., circulator 104) configured to use SF₆ at about 2 atm to act as an insulator to reduce arcs within the circulator 904. However, different from FIG. 1, RF system 900 includes third and fourth waveguides 901c and 901d coupled between the circulator 904 and the second and third waveguides 902b and 902c, respectively.

Except for the circulator 904, RF system 900 may be configured to operate using a non-reactive gas (e.g., N₂) other than SF₆ at about 2 atm. For example, non-reactive gas supply 906a may supply a non-reactive gas other than SF₆. At least one first regulator 912a may be connected to at least one of first and/or second waveguide 902a and 902b, and at least one first regulator 912a may be connected to third waveguide 902c. First regulators 912a may be configured to maintain a non-reactive gas other than SF₆ at a pressure of about 2 atm in the RF system 900, except for the circulator 904. However, example embodiments are not limited thereto. For example, the first regulators 912a may be configured to maintain the pressure of the N₂ gas in the RF system 900 from the third RF window 901c to the second RF window 901b at about 2 atm, and to maintain the pressure of the N₂ gas from the first RF window 901a to the fourth RF window 901d at a higher pressure (e.g., about 3 atm to about 4 atm) to account for double strength electric fields from reflected RF pulses that may be present in this section of the RF system 900.

Non-reactive gas supply 906b may supply SF₆. At least one second regulator 912b may be connected to a waveguide of the circulator 904. Regulator 912b may be configured to maintain SF₆ at a pressure of about 2 atm in the circulator 904.

Accordingly, RF system 900 according to example embodiments may reduce or prevent arcing inside circulator 904 while greatly reducing an amount of SF₆ used in the system. For example, a volume of SF₆ used by RF system 900 may be about 5% (e.g., about 5-10%) of a volume of SF₆ used in current RF systems (e.g., RF system 100), This may therefore lower greenhouse gas emissions by about 95% (e.g., about 90-95%).

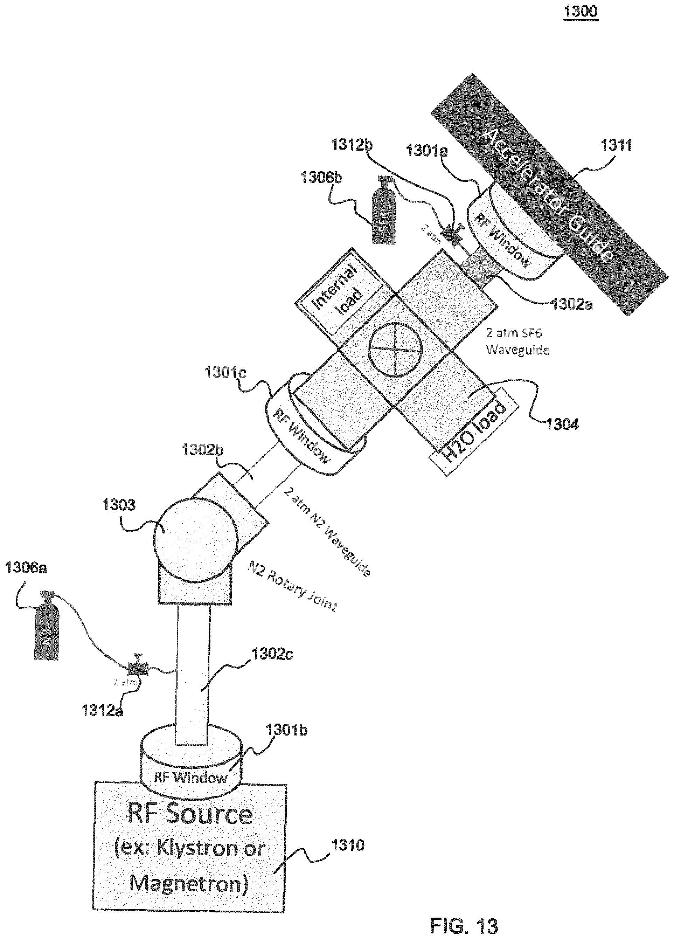

FIG. 13 is a RF system according to some example embodiments.

Referring to FIG. 13, a RF system 1300 according to example embodiments may include first to third RF windows 1301a to 1301c, first to third waveguides 1302a to 1302c, a rotary joint 1303, a circulator 1304, first and second non-reactive gas supplies 1306a and 1306b, first and second regulators 1312a and 1312b, a RF source 1310, and/or an accelerator guide 1311. As used herein the waveguides 1302a to 1302c may be referred to as waveguides, waveguide portions, or portions. As used herein, the circulator 1304 may be referred to as a component. Descriptions of FIG. 13 similar to FIGS. 1-3, 5-7, and 9 will be omitted and the differences will be mainly described.

More specifically, the RF system 1300 includes the first RF window 1301a coupled between the accelerator guide 1311 and the first waveguide 1302a, the circulator 1304 coupled between the first waveguide 1302a and the third RF window 1301c, the second waveguide 1302b coupled between the third RF window 1301c and the rotary joint 1303, and the third waveguide 1302c coupled between the rotary joint 1303 and the second RF window 1301b, which is coupled between the third waveguide 1302c and the RF source 1310.

The circulator 1304 may be an existing circulator (e.g., circulator 104) configured to use SF₆ gas at about 2 atm to act as an insulator to reduce arcs within the circulator 1304. However, different from FIG. 9, the location of the circulator 1304 may be moved closer to the accelerator guide 1311. For example, as may be appreciated from FIG. 13, the only waveguide portion between the circulator 1304 and the accelerator guide 1311 may be the first waveguide 1302a.

Therefore, as discussed above, the circulator 1304 may reduce or prevent reflected waves from the accelerator guide 1311 from reaching any of the second waveguide 1302b, the rotary joint 1303, the third waveguide 1302c, or the RF source 1310.

As shown in FIG. 13, the circulator 1304 and the first waveguide 1302a may be configured to operate using SF₆ gas at about 2 atm. For example, the non-reactive gas supply 1306b may supply SF₆ gas, and at least one regulator 1312b may be configured to maintain the SF₆ gas in the first waveguide 1302a and the circulator 1304 at about 2 atm. Similar to the RF system 100 shown in FIG. 1, SF₆ maintained at a pressure of about 2 atm may be sufficient to reduce or prevent arcing in the waveguide between the accelerator guide 1311 and the circulator 1304, where the double strength electric fields from reflected RF pulses may be present.

As the circulator may reduce and/or prevent the double strength electric fields from reaching the waveguides 1302b-1302c and the rotary joint 1303, a non-reactive gas other than SF₆ (e.g., N₂ gas) maintained at a pressure of about 2 atm may be sufficient to reduce or prevent arcing in this section of the RF system 1300. Therefore, non-reactive gas supply 1306a may supply a non-reactive gas different from SF₆ (e.g., N₂ gas), and at least one regulator 1312a may be configured to maintain the non-reactive gas in the waveguide second waveguide 1302b, the third waveguide 1303c, and the rotary joint 1303 at about 2 atm.

Accordingly, RF system 1300, according to example embodiments, may reduce or prevent arcing inside circulator 1304 while greatly reducing an amount of SF₆ used in the system. For example, a volume of SF₆ used by RF system 1300 may be about 5% (e.g., about 5-10%) of a volume of SF₆ used in current RF systems (e.g., RF system 100), This may therefore lower greenhouse gas emissions by about 95% (e.g., about 90-95%).Although the present invention has been described in detail with reference to example embodiments, the present invention is not limited by the disclosed examples from which the skilled person is able to derive other variations without departing from the scope of the invention.

None of the elements recited in the claims are intended to be a means-plus-function element within the meaning of 35 U.S.C. §112(f) unless an element is expressly recited using the phrase "means for" or, in the case of a method claim, using the phrases "operation for" or "step for."

Example embodiments being thus described, it will be obvious that the same may be varied in many ways. Such variations are not to be regarded as a departure from the spirit and scope of the present invention, and all such modifications as would be obvious to one skilled in the art are intended to be included within the scope of the following claims.

The terminology used herein is for the purpose of describing particular embodiments only and is not intended to be limiting of example embodiments. As used herein, the singular forms "a," "an," and "the," are intended to include the plural forms as well, unless the context clearly indicates otherwise. As used herein, the terms "and/or" and "at least one of" include any and all combinations of one or more of the associated listed items. It will be further understood that the terms "comprises," "comprising," "includes," and/or "including," when used herein, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items. Expressions such as "at least one of," when preceding a list of elements, modify the entire list of elements and do not modify the individual elements of the list.

It should also be noted that in some alternative implementations, the functions/acts noted may occur out of the order noted in the figures. For example, two figures shown in succession may in fact be executed substantially concurrently or may sometimes be executed in the reverse order, depending upon the functionality/acts involved.

Specific structural and functional details disclosed herein are merely representative for purposes of describing example embodiments. This invention may, however, be embodied in many alternate forms and should not be construed as limited to only the embodiments set forth herein.

### NON-LIMITING ILLUSTRATIVE EMBODIMENTS

The following is a list of non-limiting illustrative embodiments disclosed herein:
Illustrative embodiment 1. A radiation frequency system for a radiation therapy machine including a first portion containing a gas, a second portion containing the gas, a component between the first portion and the second portion, and a vacuum pump configured to generate a vacuum state inside the component.
Illustrative embodiment 2. The radio frequency system of illustrative embodiment 1, wherein the gas is a non-reactive gas.
Illustrative embodiment 3. The radio frequency system of any of the preceding illustrative embodiments, wherein the gas is N₂.
Illustrative embodiment 4. The radio frequency system of any of the preceding illustrative embodiments, further including a regulator configured to maintain a pressure of the gas at 1 atm to 2 atm in the first portion and the second portion.
Illustrative embodiment 5. The radio frequency system of any of the preceding illustrative embodiments, wherein the component is a circulator.
Illustrative embodiment 6. The radio frequency system of any of the preceding illustrative embodiments, wherein the component includes a plurality of openings, and wherein the vacuum pump is configured to generate the vacuum state inside the component through the plurality of openings.
Illustrative embodiment 7. The radio frequency system of illustrative embodiment 6, wherein at least one of the first portion or the second portion includes a waveguide, and wherein each of the plurality of openings is smaller than a wavelength of a radio frequency signal of the waveguide.
Illustrative embodiment 8. The radio frequency system of illustrative embodiment 6 or illustrative embodiment 7, wherein the plurality of openings are slits.
Illustrative embodiment 9. The radio frequency system of any of the preceding illustrative embodiments, wherein the vacuum pump is attached to the component with conflat flanges.
Illustrative embodiment 10. The radio frequency system of any of the preceding illustrative embodiments, wherein the vacuum pump is configured to generate the vacuum state to act as an insulator to reduce arcs within the radio frequency system.
Illustrative embodiment 11. The radio frequency system of any of the preceding illustrative embodiments, further including a first radio frequency window between the first portion and the component, and a second radio frequency window between the second portion and the component.
Illustrative embodiment 12. A radio frequency system for a radiation therapy machine, the radio frequency system including a first portion containing a gas at a first pressure, a second portion containing the gas, and a component between the first portion and the second portion, the component containing the gas at a second pressure, the second pressure being different from the first pressure.
Illustrative embodiment 13. The radio frequency system of illustrative embodiment 12, wherein the first pressure is 1 atm to 3 atm and the second pressure is 3 atm to 5 atm.
Illustrative embodiment 14. The radio frequency system of illustrative embodiment 12, wherein the first pressure is 3 atm to 5 atm and the second pressure is 5 atm to 7 atm.
Illustrative embodiment 15. The radio frequency system of any of illustrative embodiments 12-14, further comprising a first radio frequency window between the first portion and the component, and a second radio frequency window between the second portion and the component.
Illustrative embodiment 16. The radio frequency system of any of illustrative embodiments 12-15, further including a first radio frequency window between the first portion and the component, and a second radio frequency window between the second portion and the component, wherein the first radio frequency window and the second radio frequency window are block windows.
Illustrative embodiment 17. The radio frequency system of illustrative embodiment 14, further including a third portion between the first portion and a rotary joint, the third portion containing the gas at a third pressure, a fourth portion between the second portion and a radio frequency source, the fourth portion containing the gas at the third pressure, a third radio frequency window between the third portion and the first portion, and a fourth radio frequency window between the second portion and the fourth portion.
Illustrative embodiment 18. The radio frequency system of illustrative embodiment 17, wherein the third pressure is 1 atm to 3 atm.
Illustrative embodiment 19. The radio frequency system of any of illustrative embodiments 12-18, wherein the component is a circulator.
Illustrative embodiment 20. The radio frequency system of any of illustrative embodiments 12-19, wherein the gas is a non-reactive gas.
Illustrative embodiment 21. The radio frequency system of any of illustrative embodiments 12-20, wherein the first pressure is lower than the second pressure.
Illustrative embodiment 22. A radio frequency system for a radiation therapy machine, the radio frequency system including, a first portion containing a first gas at a first pressure, a second portion containing the first gas, a component between the first portion and the second portion, the component containing a second gas at a second pressure, the second gas being different from the first gas.
Illustrative embodiment 23. The radio frequency system of illustrative embodiment 22, wherein the first pressure is the same as the second pressure.
Illustrative embodiment 24. The radio frequency system of any of illustrative embodiments 22-23, wherein the second gas is SF₆ and the first gas is a non-reactive gas different from SF₆.
Illustrative embodiment 25. The radio frequency system of illustrative embodiment 24, wherein the first gas is N₂.
Illustrative embodiment 26. The radio frequency system of any of illustrative embodiments 22-25, further including a first radio frequency window between the first portion and the component, and a second radio frequency window between the second portion and the component.
Illustrative embodiment 27. A radio frequency system for a radiation therapy machine, the radio frequency system including a first portion containing a first gas at a first pressure, a second portion containing a second gas at a second pressure, the second gas being different from the first gas, and a component between the first portion and the second portion, the component containing the first gas at the first pressure.
Illustrative embodiment 28. The radio frequency system of illustrative embodiment 27, wherein the first pressure is the same as the second pressure.
Illustrative embodiment 29. The radio frequency system of any of illustrative embodiments 27-28, wherein the first gas is SF₆ and the second gas is a non-reactive gas different from SF₆.
Illustrative embodiment 30. The radio frequency system of illustrative embodiment 29, wherein the second gas is N₂.
Illustrative embodiment 31. The radio frequency system of any of illustrative embodiments 27-30, further including a rotary joint, and a RF source, wherein the component is between the rotary joint and the RF source.

## Claims

1. A radio frequency system (300, 500, 600, 700, 900, 1300) for a radiation therapy machine, the radio frequency system comprising:
a first portion containing a gas;
a second portion containing the gas;
a component (304, 504, 604, 704, 904, 1304) between the first portion and the second portion; and
a vacuum pump (305) configured to generate a vacuum state inside the component, optionally the gas is a non-reactive gas, optionally the gas is N₂, optionally the radio frequency system further comprising:
a regulator (312, 512a-b, 612a-c, 712a-b, 912a-b, 1312a-b) configured to maintain a pressure of the gas at 1 atm to 2 atm in the first portion and the second portion.

2. The radio frequency system of claim 1, wherein the component includes a plurality of openings (306b1, 506b), and
wherein the vacuum pump is configured to generate the vacuum state inside the component through the plurality of openings, optionally at least one of the first portion or the second portion includes a waveguide (302a-c, 304a, 502a-c, 504a, 602a-c, 702a-c, 800, 902a-c, 1302a-c), and
wherein each of the plurality of openings is smaller than a wavelength of a radio frequency signal of the waveguide, optionally the plurality of openings are slits.

3. The radio frequency system of claim 1 or 2, wherein the vacuum pump is attached to the component with conflat flanges.

4. A radio frequency system for a radiation therapy machine (300, 500, 600, 700, 900, 1300), the radio frequency system comprising:
a first portion containing a gas at a first pressure;
a second portion containing the gas; and
a component (304, 504, 604, 704, 904, 1304) between the first portion and the second portion, the component containing the gas at a second pressure, the second pressure being different from the first pressure, optionally the gas is a non-reactive gas and optionally the first pressure is lower than the second pressure, optionally the first pressure is 3 atm to 5 atm and the second pressure is 5 atm to 7 atm wherein optionally the first pressure is 1 atm to 3 atm and the second pressure is 3 atm to 5 atm.

5. The radio frequency system of any one of the preceding claims, further comprising:
a first radio frequency window (301a, 501a) between the first portion and the component (304, 504); and
a second radio frequency window (301b, 501b) between the second portion and the component (504).

6. The radio frequency system of claim 4 or 5, further comprising:
a first radio frequency window between the first portion and the component; and
a second radio frequency window between the second portion and the component,
wherein the first radio frequency window and the second radio frequency window are block windows.

7. The radio frequency system of claim 4, 5 or 6, further comprising:
a third portion between the first portion and a rotary joint (503), the third portion containing the gas at a third pressure;
a fourth portion between the second portion and a radio frequency source (510), the fourth portion containing the gas at the third pressure;
a third radio frequency window (501c) between the third portion and the first portion; and
a fourth radio frequency window (501d) between the second portion and the fourth portion, optionally the third pressure is 1 atm to 3 atm.

8. A radio frequency system for a radiation therapy machine, the radio frequency system comprising:
a first portion containing a first gas (906a, 1306a) at a first pressure;
a second portion containing the first gas (906a, 1306a); and
a component (904, 1304) between the first portion and the second portion, the component containing a second gas (906b, 1306b) at a second pressure, the second gas (906b, 1306b) being different from the first gas (906a, 1306a), optionally the first pressure is the same as the second pressure, optionally the second gas is SF₆ and the first gas is a non-reactive gas different from SF₆, optionally the first gas is N₂.

9. The radio frequency system of claim 8, further comprising:
a first radio frequency window (901a, 1301a) between the first portion and the component (904, 1304); and
a second radio frequency window (901b, 1301b) between the second portion and the component (904, 1304).

10. A radio frequency system for a radiation therapy machine, the radio frequency system comprising:
a first portion containing a first gas (906a, 1306a) at a first pressure;
a second portion containing a second gas (906b, 1306b) at a second pressure, the second gas being different from the first gas; and
a component (904, 1304) between the first portion and the second portion, the component containing the first gas at the first pressure, optionally the first pressure is the same as the second pressure, optionally the first gas is SF₆ and the second gas is a non-reactive gas different from SF₆, optionally the second gas is N₂.

11. The radio frequency system of any one of the preceding claims, further comprising:
a rotary joint (303, 503, 603, 703, 903, 1303); and
a RF source (310, 510, 610, 710, 910, 1310),
wherein the component (304, 504, 604, 704, 904, 1304) is between the rotary joint and the RF source.

12. The radio frequency system of any one of the preceding claims, wherein the component is a circulator (304, 504, 604, 704, 904, 1304).
